# EUROPEAN PATENT SPECIFICATION

(11) **EP 2 553 470 B1**
(45) Date of publication and mention of the grant of the patent: **11.05.2016**
(21) Application number: 10711886.1
(22) Date of filing: 30.03.2010
(51) Int. Cl.: G01N 33/86, G01N 33/543, G01N 33/49, C12Q 1/56

(54) **COMPOSITION FOR THE DETERMINATION OF COAGULATION CHARACTERISTICS OF A TEST LIQUID**
DIAGNOSTISCHE ZUSAMMENSETZUNG UND SEINE VERWENDUNG BEI DER BESTIMMUNG VON GERINNUNGSEIGENSCHAFTEN EINER TESTFLÜSSIGKEIT
COMPOSITION DE DIAGNOSTIC ET SON UTILISATION POUR LA DÉTERMINATION DES CARACTÉRISTIQUES DE COAGULATION D'UN LIQUIDE D'ESSAI

(43) Date of publication of application: 06.02.2013
(60) Divisional application: 15194046.7
(73) Proprietor: C A Casyso AG, 4132 Muttenz (CH)
(72) Inventor: SCHUBERT, Axel, 81927 Muenchen (DE)
(74) Representative: Isarpatent
(86) International application number: PCT/EP2010/054161
(87) International publication number: WO 2011/120556

(56) References cited:
- EP-A1- 1 234 614
- EP-A1- 2 040 073
- WO-A1-2008/093216
- WO-A2-2009/115608
- US-A- 4 755 461

## Description

### FIELD OF THE INVENTION

The present invention is directed to diagnostic compositions for use in the viscoelastic analysis of a test liquid, and to a container comprising same. The present invention further is directed to a method of performing a viscoelastic analysis on a blood sample, and to the use of the diagnostic composition in such a method.

### BACKGROUND

The coagulation of blood is a complex process during which blood forms solid clots. It is an important part of hemostasis (the cessation of blood loss from a damaged vessel) whereby a damaged blood vessel wall is covered by a blood clot to stop hemorrhage and aid repair of the damaged vessel. Disorders in coagulation can lead to increased hemorrhage and/or thrombosis and embolism.

In a normal individual, coagulation is initiated within 20 seconds after an injury occurs to the blood vessel damaging the endothelial cells. Platelets immediately form a haemostatic plug at the site of injury. This process is called primary haemostasis. Secondary haemostasis follows if plasma components called coagulation factors respond in a complex cascade to form fibrin strands which strengthen the platelet plug.

The coagulation cascade of secondary hemostasis has two pathways, the Contact Activation pathway (formerly known as the Intrinsic Pathway) and the Tissue Factor pathway (formerly known as the Extrinsic pathway) that lead to fibrin formation. It was previously thought that the coagulation cascade consisted of two pathways of equal importance joined to a common pathway. It is now known that the primary pathway for the initiation of blood coagulation is the Tissue Factor pathway. The pathways are a series of reactions, in which a zymogen of a serene protease and its glycoprotein co-factor are activated to become active components that then catalyze the next reaction in the cascade. Coagulation factors are generally indicated by Roman numerals from I - XIII, with a lowercase 'a' appended to indicate the activated form.

Fibrinolysis is the process where the fibrin clot is broken down. Tissue plasminogen activator (tPA) and urokinase are the agents that convert plasminogen to active plasmin, thus allowing fibrinolysis to occur.

The detection of normal or decreased functionality of these coagulation components is important in order to assess patients' hemostasis disorders.

Several methods of measuring the coagulation characteristics of blood are known. Some such devices attempt to simulate the natural flow of blood in the veins and arteries of a living subject, while other measurement techniques are performed in static blood volumes.

An accurate measurement of the ability of a patient's blood to coagulate in a timely and effective fashion is crucial to certain surgical and medical procedures. Rapid and accurate detection of abnormal coagulations is also of particular importance with respect to appropriate treatment to be given to patients suffering from clotting disorders. Often the condition of such patients makes it necessary to administer blood components, anti-coagulants, certain fibrinolytic agents, anti-platelet agents, or compounds inducing the reverse effects of said agents. In these cases, the treatment dose can be adapted to the extent of a clotting disorder previously determined.

Measurements of blood clotting are provided by various devices, for example as disclosed in (US 5,777,215), (US 6,537,819), or (US 5,777,215). These devices measure the mechanical properties of the clot throughout its structural development. These systems are summarized under the term "viscoelastic methods", as they continuously detect viscoelastic properties of the blood clot while its formation and lysis.

A viscoelastic measurement provides information about several distinct parameters, for example the time between coagulation activation and clot initiation (clotting time CT), the dynamics of clot formation (clot formation time CFT), the firmness of the clot (amplitudes A5-A30 and maximum clot firmness MCF), or the extent of fibrinolysis (maximum lysis ML).

A number of references describe instruments for measuring blood clotting characteristics based upon mechanical movements. These instruments monitor the elastic properties of blood as it is induced to clot under a low shear environment, i.e. in static blood volumes. The patterns of change in shear elasticity enable the determination of the kinetics of clot formation, as well as the strength and stability of the formed clot. The strength and stability of the clot provide information about the ability of the clot to perform the "work of hemostasis" (i.e., stop or prevent abnormal bleeding) and about the adequacy of blood platelet-fibrin interaction. The kinetics of clot formation mainly provides information about the functionality of coagulation factors. Analysis of all of this information provides results which are useful to predict bleeding, to monitor and manage thrombosis, or to monitor fibrinolysis.

However, as the clotting process consists of various interlinked components, the use of specific activators and inhibitors is further applied in order to detect hemostasis disorders more specifically.

Accordingly, the reagents used in viscoelastic analysis consist of an initial activator (e.g., an activator of either the intrinsic or the extrinsic pathway) and optionally one or more inhibitors (e.g., fibrinolysis inhibitors, heparin inhibitors, platelet inhibitors) and/or one or more further specific factor of the coagulation cascade.

Optionally further components may be added:
- Calcium (CaCl₂): The calcium is added for recalcification of the sample. Blood samples can be prevented from clotting by several different anticoagulatory substances like heparin, EDTA, citrate. Typically functional tests are done with blood anticoagulated with citrate. Citrate moderately complexes calcium of the blood sample. Calcium is necessary for the coagulation process, it is involved in complex formation and is co-factor for most of the coagulation factors (e.g., FI, FII, FV, FVII, FVIII, FIX, FX, FXI, FXIII, TF). Therefore, recalcification of the sample is necessary to ensure correct coagulation in the sample, if the sample was citrated during blood withdrawal (by using a blood tube containing citrate).
- Phospholipids: Several complexes in the coagulation cascade are phospholipid-dependent and, therefore, additional phospholipids might be added.
- Stabilizers: For the stabilization of the reagents between the time of production and the analysis (e.g. albumin, gelatine)

Depending on the diagnostic aim, these reagents can be used either alone or in combination: For example, a measurement with only intrinsic activator in the sample can be combined with a measurement with intrinsic activator and a sufficient amount of heparin inhibitor (e.g., heparinase) in the sample to detect the presence of heparin in the test liquid; a combination of extrinsic activator and platelet inhibitor (e.g., Cytochalasin D) in the sample is applied to determine the activity of fibrinogen without platelet contribution in the test liquid.

There is a reagent concept for viscoelastic measurements in the literature (ReoPro-modified TEG: Wenker et al.: Thrombelastography, The Internet Journal of Anesthesiology, 2000, Volume 1 Number 3; (http://www.ispub.com/ostialindex.php?xmlFilePath=journals/ija/ volln3/teg.xml); Ruttmann et al.: Hemodilution Enhanced Coagulation Is Not Due to Platelet Clumping, Anesthesiology 2004; 101: A150; Recombiplastin- and ReoPro-modified TEG:http://www.transfusionguidelines.org.uk/ docs/pdfs/bbt_app-use_teg-sop-example.pdf; TF- and Trasylol-modified TEG: Tanaka et al.: Evaluation of a novel kallikrein inhibitor on hemostatic activation in vitro, Thrombosis Research, Volume 113, Issue 5 , 2004, Pages 333-339) that is based on the combination of commercially available activator reagents intended for other tests, such as the prothrombin time activator Innovin or Recombiplastin®, combined with customer-made CaCl2 solution and drugs, such as ReoPro® (abciximab) and Trasylol® (aprotinin). This leads to a low standardization, many pipetting steps and many sources of error.

There is a reagent system for viscoelastic measurements marketed by Pentapharm, which is based on standardized reagents, most of which are provided to the customer in a liquid form, which are pipetted by the user into the test cup using standardized operating procedures. This standardizes the application; however it still requires several pipetting steps for the analysis. For example, to perform a FIBTEM test together with an EXTEM control test, the pipetting of blood, CaCl₂ solution, extrinsic activator and a platelet inhibitor may result in the performance of a total of 8 pipetting steps (including three times changing of the tip during one test procedure) and the need for 3 different reagents that have to be handled by the user. This provides a requirement for training consumes time and is a potential source of error. There are other reagent systems on the market, which are based on a variety of reagents. Some of them are liquid, and have to be pipetted into the cup (e.g. CaCl₂ solution), some are dried into the test cup (such as heparinase) and some are provided in small vials, in a quantity intended for one test. A characteristic of these reagents is that still each reagent is typically provided alone, and therefore several steps are required at least for tests requiring more than one active reagent.

Accordingly, some efforts have been done in the past to provide a simpler reagent system for viscoelastic measurements of blood or blood components:
- drying them directly into the cup which takes the volume of the sample during measurement
- compound stable liquid combinations of the reagents in the working concentration

One shortcoming of the strategy of drying active reagents directly into the test cup is that these are typically made of plastic and are light, which makes the filling of these cups in automated reagent dispensing lines more difficult. This makes manual filling steps necessary or the development of specialized equipment, which are both costly. Another shortcoming of this strategy is that the active components or stabilizers may interfere with the adhesion strength of the blood clot on the cup surfaces, which is required to perform correct measurements. For example, there has been shown diminished clot adhesion after incubation of albumin solution into the cup (Albumin is a typical stabilizer used to stabilize all kinds of proteins in reagents):

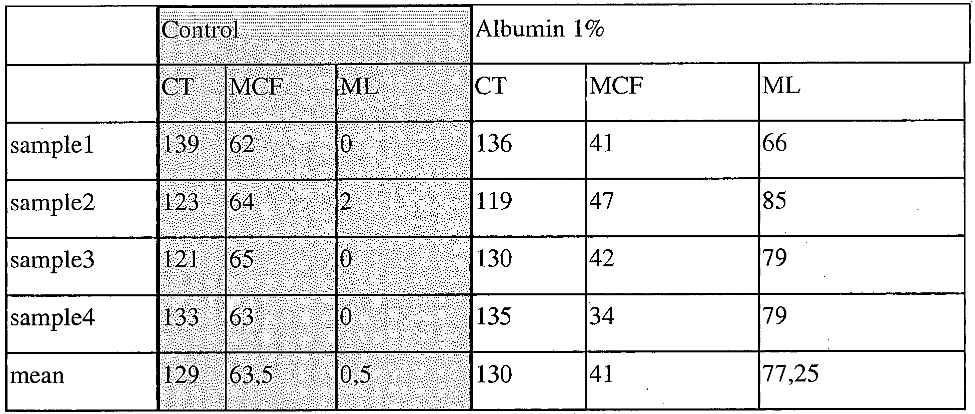

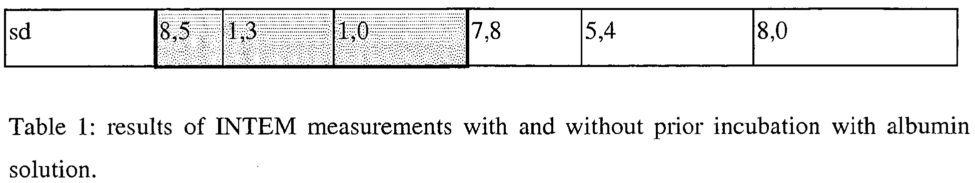

Another possible strategy to simplify the handling of the reagents is to combine the different reagents necessary for one test in liquid phase in their working concentration. The main problem here is the interaction of the different substances while staying together for a longer period. Some components negatively affect the stability of each other when staying together in the liquid phase at higher concentrations; for example, CaCl₂ disturbs the stability of Tissue Factor reagent in liquid phase over the time.

Moreover, if these combined reagents should be provided in an amount sufficient for exactly one test, another problem would arise: In this case, the very small portion of liquid reagent might stick to parts of the reagent container or the cap and might thus not mix sufficiently with the test liquid when the analysis is performed.

One strategy proposed to solve the named aspects was disclosed by Kolde et al. in US patent application 20040071604. In this application, a cup system for viscoelastic analyses is presented, in which the lower end of the cup is divided in several reagent chambers. This allows to place the reagents independently into the different chambers, without mixing them and then to freeze-dry the reagents.

However, disadvantages of this solution include the need for a very precise pipetting process, as the separate reagent chambers are very small and also the problem that the reagent drops might still mix before the freeze-drying by vibrations present on the reagent filling line. Another problem is the possible air-drying of the small reagent drops during the processing under room conditions before the lyophilisation process starts. Again the problem of automatically handling the small plastic and thus very light using standard reagent-filling lines is present.

The most coagulation test methods in routine lab use just measure the time from adding an activator to the sample until the first initial formation of a fibrin clot can be detected (= clotting time). They stop at this point and no further measurement is done. This has the implications, that in these methods a firm adhesion of the blood to the surfaces of the measurement cell is not necessary. Accordingly, the variety of analyzers and reagents available for the assessment of clotting times with such methods does not have to handle the unique problems connected with viscoelastic measurements.

EP 0 972 203 describes a reagent for determining aPTT, comprising at least one coagulation protein, phospholipids and an activator of intrinsic coagulation in co-lyophilized form. However, here the problem arises that all components required for performing the analysis are in immediate contact with each other. Therefore, problems regarding long-term stability or incompatibilities may occur by using substances in this form.

WO 2008/093216 describes a diagnostic composition for use in the viscoelastic analysis of a test liquid and to a container comprising same. The composition comprises at least an activator of coagulation, and, optionally, CaCl₂ and one or more inhibitors and/or coagulation components. The composition is present as an essentially dry mixture of all constituents and in an amount sufficient for performing one single viscoelastic analysis of a specified blood or plasma sample. However, it is not disclosed that the constituents are each incorporated in spatially separated pellets.

### SUMMARY OF THE INVENTION

Therefore, it is an object of the present invention to provide a diagnostic composition, which allows for a safe, reproducible and easy to use procedure for different tests in viscoelastic systems. It is a further object of the present invention to provide a diagnostic composition which is specifically adapted to one single analysis of a blood sample and has a superior reagent stability regarding prior art compositions. It is a still further object of the present invention to provide a diagnostic method which provides reliable and reproducible results, is easy to handle and which provides a standardized system for the determination of the coagulation characteristics of a blood sample. A further object of the invention is to provide a diagnostic composition of the above kind, having an improved long-term stability.

These objects are attained by the method of claim 1, the composition of claim 6, and the container of claim 13.

Preferred embodiments are set forth in the dependent claims.

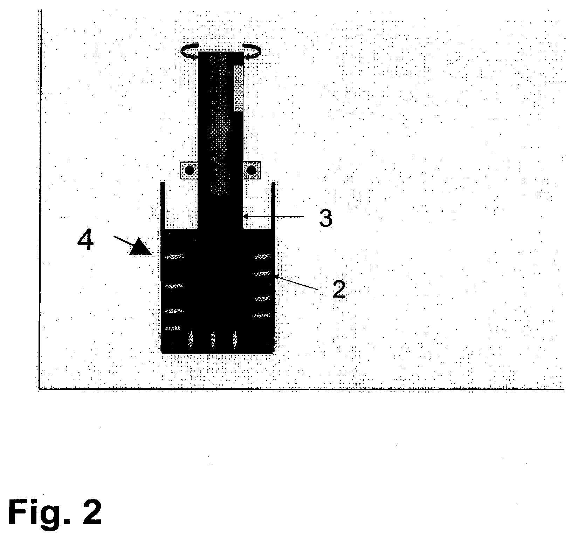

By using the diagnostic composition of the invention, the tests may be performed as follows: a defined volume of a sample (e.g., whole blood, blood plasma etc.) is added directly into a container 1 containing the diagnostic composition. After dissolving of the composition in the blood sample, the resulting mixture is pipetted from the container 1 into the measuring cup 2 of a measuring apparatus 4. The cup 2 is then put into a position such that the pin 3 is immersed into the liquid in the test cup (cp. Fig. 2).

Therefore, the user needs only 4 pipetting steps for each test to perform (in the prior art liquid system up to 8 steps, see above) and no change of the pipette tip is necessary.

Thus, it is clear that the present system for the determination of coagulation characteristics of a blood sample can be handled easier, thereby making the likelihood of errors smaller, which can be due to an imprecise line of action by a (potentially less experienced) operator.

According to the invention, the constituents are not present in a substance mixture, but are each incorporated in spatially separated pellets. This allows for an improved long-therm stability of the composition without deteriorating its other characteristics.

Therefrom, further advantages may arise as, for example, a higher reproducibility of the results to be achieved, and thus, a higher degree of standardization.

### BRIEF DESCRIPTIONS OF THE DRAWINGS

Further features and advantages of the present invention will be evident from a description of embodiments with reference to the figures.

In the figures:

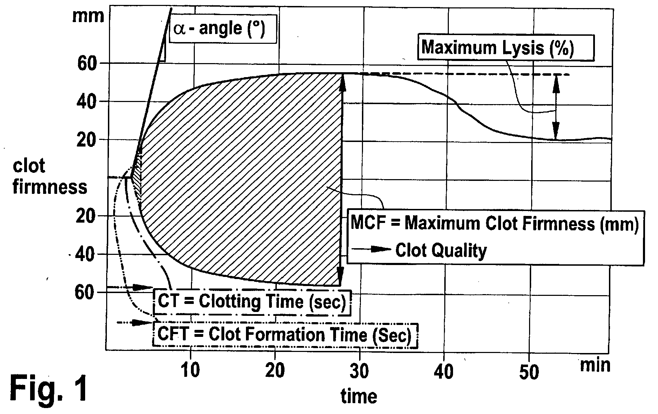

Figure 1 is an exemplary diagram showing a typical thrombo-elastometric measurement;
Figure 2 is showing a measuring apparatus 4 for thromboelastometric analysis;
Figure 3 is an illustration of a measuring cup 2 of a measuring apparatus 4 of the prior art;
Figure 4A, B, C are schematic cross-sectional views of three preferred embodiments of a container 1 of the present invention.

### DETAILED DESCRIPTION OF THE INVENTION

In one aspect, the present invention provides a diagnostic composition for use in the viscoelastic analysis of a test liquid, comprising:
a) at least one activator of coagulation; and
b) a calcium salt, preferably CaCl₂, in an amount sufficient to ensure recalcification of the test liquid; and,
c) a heparin inhibitor;
characterized in that
the constituents are present in an essentially dry form and in an amount sufficient for performing one single viscoelastic analysis of a specified test liquid,
and wherein the constituents are not present in a substance mixture, but are each incorporated in spatially separated pellets.

The calcium salt preferably is CaCl₂.

The diagnostic composition or reaction mixture of the present invention comprises constituents, which are per se known in the art. One difference to the prior art approaches is, however, that the mixture of these constituents is provided in an essentially dry form.

"Essentially" dry in the context of the invention means a state, wherein the mixture is essentially free from any liquid or moisture, in particular being depleted of water. Water or any other liquid, however, may be present as residue in the mixture, but only to an extent, which does not negatively influence the stability of the overall composition. In particular, it has to be excluded that an interaction occurs between the different constituents, which negatively affects the stability. A remaining amount of liquid, preferably water, in the composition of up to 10 % by weight should be acceptable.

The amount sufficient for performing one single viscoelastic analysis of a specified test liquid, for example a blood sample, is that amount of all constituents in mixture, which provides the required concentration of the reagents in the final analysis of the coagulation characteristics of the blood sample, i.e. in the cup 2 of a measuring apparatus 4. Therefore, it is not necessary to further portion the diagnostic composition before or after dissolving it in a liquid.

Further, this is achieved by dissolving the substances in the test liquid (blood sample etc.) itself, not by dissolving the constituents in an amount of liquid diluent leading to the final working concentration.

It is an important element of the present invention that the constituents are not present in a substance mixture, but in a spatially separated form. This avoids stability related problems.

The spatial separation of the constituents is realized by incorporating each constituent into a separate carrier material. This term means that the constituents are each incorporated in spatially separated entities such as pellets, minitablets etc. The term "separate carrier material" does not necessarily mean that the materials used are chemically different. It is only meant that they are spatially separated from each other.

The carrier takes the form of pellets. Those pellets may be produced by methods which are per se known in the prior art. However, it is preferred to use one of the both methods of producing pellets, which will be shortly outlined in the following:
a) The respective constituent in solution is mixed with a suitable carrier material, which mixture is then converted into spray form. This spray, containing droplets of the mixture, is shock frozen in liquid nitrogen, will be warmed then under a protective atmosphere and is stored in an air tight container (such as a vial).
b) Pellets are produced such as in a), however, the starting mixture does not contain any constituent. The constituent is provided as a solution and is allowed to drop onto the pellets under a protective atmosphere. After being dried onto the pellets, the pellets are packaged in an air-tight container.

In a preferred embodiment, the carrier material comprises at least a carbohydrate, e.g. saccharose, lactose or cellulose. The carrier material preferably shows no significant influence on the clotting behaviour, the clot formation behaviour, or the clot lysis behaviour.

The activator of coagulation as mentioned above preferably is an intrinsic and/or extrinsic activator.

The extrinsic activator of coagulation in turn preferably is the Tissue Factor (TF) and is more preferably selected from lipidated TF or rTF.

The intrinsic activator of coagulation is preferably selected from celite, ellagic acid, sulfatit, kaolin, silica, RNA, or mixtures thereof.

As a second feature, the diagnostic composition of the present invention comprises a calcium salt such as CaCl₂, wherein CaCl₂ is preferably present in an amount of about 1-100 µmol/ml of test liquid. As mentioned above, the amount of CaCl₂ must be sufficient to ensure recalcification of the test liquid, in particular of the blood sample, if the sample was decalcified before. It turned out that the amount of from 3-30 µmol/ml is optimal to achieve this requirement. In order to determine the required amount of CaCl₂ to be contained in the diagnostic composition even more precisely, the exact volume of the test liquid to be collected from the patient has to be known as well as the amount of decalcifying reagent employed.

The diagnostic composition of the present invention contains aa heparin inhibitor.

Inhibitors may be used and combined depending on the precise diagnostic demands, for example, the platelet inhibitor may be a cyto-skeletton inhibitor or a GPIIb/IIIa antagonist. The like, the fibrinolysis inhibitor can be selected, for example, from aprotinine, tranexamic acid, or eaca; the heparin inhibitor might be selected, for example, from hepalinase, protamine or protamine-related peptides; and the coagulation factor can be selected, for example, from one or more coagulation factors or activated coagulation factors preferably FXa or FVa or activated protein C or FVIIa. However, it is noted that this is only a preferred selection and further inhibitors can be used if required.

In a preferred embodiment, the diagnostic composition may also contain one or more stabilizers, wherein the stabilizer preferably is albumin or gelatine.

In a preferred embodiment, the diagnostic composition may also contain one or more phospholipids, wherein the phospholipids may be a composition of different phospholipids like phosphatidyserine, phosphatidylethanolamine and phosphatidylethanolcholine. For example, mixtures of phospholipids extracted from rabbit brain may be used.

The present diagnostic composition may have the following constitution in preferred embodiments:
- extrinsic activation insensitive for heparin: Combination of extrinsic activator, heparin inhibitor and stabilizer and CaCl₂
- intrinsic activation insensitive for heparin: Combination of intrinsic activator, heparin inhibitor and stabilizer and CaCl₂
- extrinsic activation without platelet activation, insensitive for heparin: Combination of extrinsic activator, platelet inhibitor, heparin inhibitor and stabilizer and CaCl₂
- intrinsic activation without platelet activation, insensitive for heparin: Combination of intrinsic activator, platelet inhibitor, heparin inhibitor and stabilizer and CaCl₂
- extrinsic activation with inhibition of fibrinolysis, insensitive for heparin: Combination of extrinsic activator, fibrinolysis inhibitor, heparin inhibitor and stabilizer and CaCl₂
- intrinsic activation with inhibition of fibrinolysis, insensitive for heparin: Combination of intrinsic activator, fibrinolysis inhibitor, heparin inhibitor and stabilizer and CaCl₂
- extrinsic activation with additional coagulation factor, insensitive for heparin: Combination of extrinsic activator, one additional coagulation factor, heparin inhibitor and stabilizer and CaCl₂
- intrinsic activation with additional coagulation factor, insensitive for heparin: Combination of intrinsic activator, one additional coagulation factor, heparin inhibitor and stabilizer and CaCl₂

In a second aspect, the present invention provides a container 1, comprising the diagnostic composition as defined above. The container 1 preferably takes the form of a vial or a cuvette.

The container 1 is formed from a material (e.g., plastic or glass) which is not corroded or otherwise affected by the reagents to be filled in or the test liquid to be filled in.

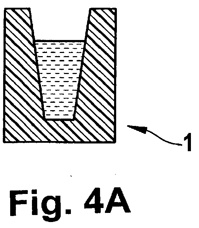

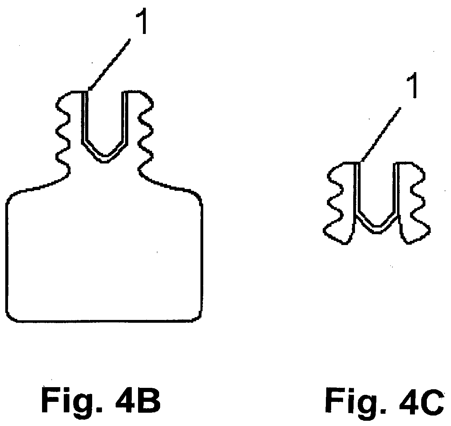

The container 1 may have cylindrical shape, but its shape does not necessarily have to be cylindrical. The container 1 may have a form which reduces its inner lateral profile from the upper opening to the bottom, as for example a conically shaped form as indicated in Fig. 4 or at least a partially conical form. This provides a better handling of the usually small amounts of liquid reagent mixture. Using flat bottom vials with a smaller diameter would reduce this problem, but such vials might then have an opening diameter which is too narrow to be handled with standard pipetting equipment used in connection with diagnostic devices like, for example, the ROTEM thrombo-elastometer. (Regarding the design of the ROTEM system and how to use it, it is referred to the publication of US 5,777,215).

Furthermore, such small vials might become harder to manage for common automated processing systems and might thus increase the production costs.

The cross-section of a basically axially symmetric container 1 of a preferred embodiment is shown in Fig. 4A. However, it should be explicitly noted that the present invention is not restricted thereto, and also U-shaped, rectangular shaped or the like forms may be used.

As mentioned above, the container 1 may be closed or sealed by a lid 5 or the like in order to avoid a loss of reagents, or an invasion of contaminants, water etc.

In a further embodiment, the container 1 is designed in a way that it can be directly used as the measuring cup 2 of the viscoelastic measuring apparatus 4. In other words, a viscolelastic analysis can be performed such that a respective container 1 is provided, the test liquid is added into the container 1, and the measurement is performed directly in the container 1. In this case, only the blood dispension into the container has to be performed as a liquid transfer step, which can be realized by using a manual pipette, an automated pipette, an automated dispenser or any other liquid transfer equipment.

In this embodiment, the container 1 might be designed by combination of two materials, e.g., glass and plastic or glass and a surface covering. In this context the part of the container made from glass does not necessarily have to clasp the entire underside of the plastic part but might be constructed according to Figure 4C.

In Figure 4B and C, a further embodiment of the invention can be seen. The container 1 (for example a cuvette) may be incorporated in a larger structure, for example a glass article, which provides some technical advantages: this set-up may provide a holding for the container.

In these embodiments, possible coagulation activation in the test liquid by the glass surface is excluded, while the superior sealing properties of the glass material when compared to plastic material are still used. The similar effect of suppressing possible coagulation activation in the test liquid by the glass surface can be realized by covering the glass surface (or at least the inner portion of the glass surface) with a layer of one or more substances that are not able to activate coagulation if they are in contact with blood or blood components.

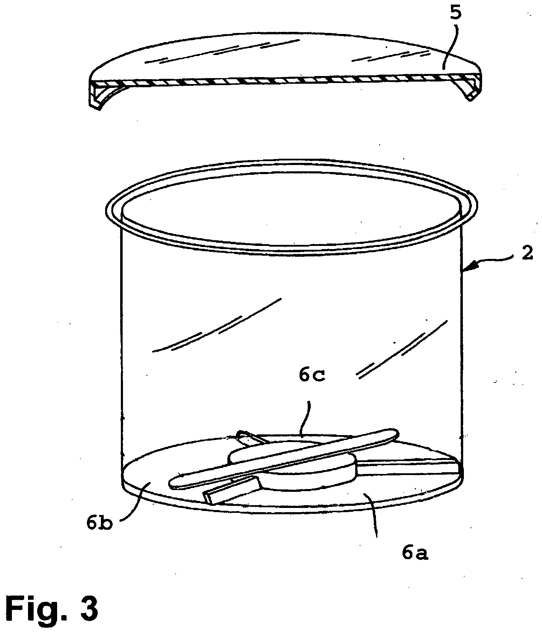

For comparison, a prior art measuring cup 2 according to US 2004/0071604 is illustrated in Fig. 3:

A container 1 is provided, which serves as reagent support and measuring vessel (i.e. can be regarded as a measuring cup 2) for analysis using various analytical processes, and has a region which is divided into at least two chambers (6a, 6b, 6c) by one or more bars extending from the container wall or the container base, wherein the chambers are arranged so that liquid or solid reagents may be introduced therein without them being able to be mixed by diffusion or running into one another. The container 1 is used for drying or freeze-drying by with completely or partly filled chambers and serves at the same time as a measuring vessel after re-dissolving the dried material by adding water, reagent solution, or the sample present in aqueous phase.

In a further aspect, the present invention is directed to an in vitro method of performing a viscoelastic analysis on a blood sample comprising the steps of:
a) providing a blood sample;
b) providing a container 1 as defined above;
c) adding the blood sample into said container 1, thereby dissolving the diagnostic composition contained therein;
d) putting the container 1 into an apparatus 4 suitable for performing a viscoelastic analysis; and
e) performing the viscoelastic analysis of said mixture.

As already mentioned above, the test liquid is a blood sample, preferably is a mammalian, more preferably a sample of human blood or blood components (e.g., whole blood or blood plasma).

Step c) of the method of the present invention preferably takes about 1-60, more preferably 2-10 sec and most preferred is about 5 sec. Following that time, the mixture of the diagnostic composition and the blood sample should be quickly transferred to the measuring cup 2 of the measuring apparatus 4. This is done in step d) by manually or automatically pipetting the mixture from the container 1 and by transferring it thereby to the apparatus 4, i.e. to the measuring cup 2 of the apparatus 4.

The apparatus 4 preferably is a device suited for viscoelastic measurements, for example devices disclosed in (US 5,777,215), (US 6,537,819), or (US 5,777,215).

One example of that apparatus 4 is shown in Fig. 2:

After the formation of the clot between cup 2 (cuvette) and pin 3, the clot itself is stretched by the movement of the pin 3 relative to the cup 2. The detection of the characteristic parameters of the clot is based on the mechanical coupling of cup 2 and pin 3 by the clot. This is only possible if the clot adheres on the surfaces of both cup 2 and pin 3. So, a firm adhesion on the surfaces of both cup 2 and pin 3 is essentially required for the viscoelastic analysis.

The method of the present invention comprises such a viscoelastic analysis of a blood sample in order to determine its coagulation characteristics, wherein such a viscoelastic analysis in the broadest sense is the measurement of a relative movement of a cuvette containing a blood sample relative to a punch. The analysis preferably comprises the determination of the clotting time, the clot formation time, and the firmness of the clot over time including fibrinolytic effects.

In practice, the following steps may be performed:
1. a defined volume of a sample (e.g., whole blood, plasma) is added directly into a vial containing the reagent composition; the measurement should start at a time close to the moment of adding the sample
2. after dissolving of the reagent mixture in the sample (5 sec.) the reagent-sample mixture is pipetted from the reagent vial into the measuring cup 2 (not necessary if the vial functions itself as measuring cup 2)
3. the cup 2 is then put into a position such that the pin 3 is immersed into the liquid contained in the test cup, the measurement continues until stopped by the user

Therefore, the user needs not more than 4 pipetting steps in total for each test to perform (compared to the up to 8 steps required for a liquid reagent system) and no change of pipette tip is necessary when preparing one test. This clearly indicates the direct benefit of the present invention for the person who is performing such tests.

In a further aspect, the present invention is directed to the use of a diagnostic composition or a container 1 as defined above in a method for analyzing the viscoelastic behaviour of a blood sample.

Although the present invention has been described in accordance with preferred embodiments, it is obvious for a person skilled in the art that modifications are possible in all embodiments.

### Reference signs

- 1: container
- 2: measuring cup
- 3: pin
- 4: measuring apparatus
- 5: lid
- 6 a,b,c: reagent chambers

## Claims

1. An in vitro method of performing a viscoelastic analysis on a blood sample, comprising the steps of:
a) providing a blood sample;
b) providing a container (1) containing a diagnostic composition comprising at least one activator of coagulation; a calcium salt, preferably CaCl₂, in an amount sufficient to ensure recalcification of the blood sample; and a heparin inhibitor; where the constituents are present in an essentially dry form and in an amount sufficient for performing one single viscoelastic analysis of a specified blood sample, and wherein the constituents are not present in a substance mixture, but are each incorporated inspatially separated pellets;
c) adding the blood sample into said container (1), thereby dissolving the diagnostic composition contained therein;
d) putting the container (1) into an apparatus (4) suitable for performing a viscolelastic analysis; and
e) performing the viscoelastic analysis of said mixture.

2. The method of claim 1, wherein the blood sample is a mammalian, more preferably a human blood sample.

3. The method of claims 1 or 2, wherein step c) takes about 1-60 sec., preferably about 2-10 sec., more preferably about 5 sec.

4. The method of one or more of the preceding claims, wherein the apparatus (4) is a thromboelastometer or a thrombelastograph.

5. The method of one or more of the preceding claims, wherein the analysis comprises the determination of the clotting time, the clot formation time, the firmness of the clot over time and/or fibrinolysis.

6. A diagnostic composition for use in the method of one or more of claims 1-5 for the viscoelastic analysis of a blood sample, comprising:
a) at least one activator of coagulation;
b) a calcium salt, preferably CaCl2, in an amount sufficient to ensure recalcification of the blood sample; and
c) a heparin inhibitor;
**characterized in that**
the constituents are present in an essentially dry form and in an amount sufficient for performing one single viscoelastic analysis of a specified blood sample, and wherein the constituents are not present in a substance mixture, but are each incorporated in spatially separated pellets.

7. The composition according to claim 6, where the spatial separation of the constituents is realized by incorporating each constituent into a separate carrier material, where the carrier material preferably comprises at least a carbohydrate, more preferably saccharose, lactose or cellulose.

8. The diagnostic composition of claim 6, wherein the activator of coagulation is an intrinsic and/or extrinsic activator.

9. The diagnostic composition of claim 8, wherein the extrinsic activator of coagulation is the Tissue Factor (TF), preferably lipidated TF or recombinant TF (rTF), or
wherein the intrinsic activator of coagulation is selected from the group consisting of celite, ellagic acid, sulfatit, kaolin, silica, RNA, or mixtures thereof.

10. The diagnostic composition of claim 6, wherein the heparin inhibitor is selected from heparinase, protamine or protamine-related peptides.

11. The diagnostic composition of one or more of claims 6-10, wherein the coagulation factor is selected from one or more coagulation factors or activated coagulation factors, preferably FXa or FVa or activated protein C or FVIIa, and/or
wherein CaCl2 is present in an amount of about 1-100 µmol/ml of the blood sample.

12. The diagnostic composition of one or more of claims 7-12, wherein the dry form is a lyophilized form, and/or
which further comprises a stabilizer, preferably albumin or gelatine.

13. A container, comprising the diagnostic composition of one or more of claims 6-12,
wherein the container (1) preferably takes the form of a vial or a cuvette, more preferably is formed in a way that the inner lateral profile reduces from the opening to the bottom.

14. The container of claim 13 with its inner portion shaped in a manner that it can be attached to a device for performing viscoelastic measurements.

## Patentansprüche

1. In-vitro-Verfahren zur Durchführung einer viskoelastischen Analyse einer Blutprobe, umfassend die folgenden Schritte:
a) Bereitstellen einer Blutprobe;
b) Bereitstellen eines Behälters (1), enthaltend eine diagnostische Zusammensetzung, die zumindest einen Aktivator der Koagulation; ein Kalziumsalz, vorzugsweise CaCl₂, in einer Menge ausreichend um die Rekalzifizierung der Blutprobe sicherzustellen; und einen Heparin-Inhibitor umfasst, wobei die Bestandteile in einer im Wesentlichen trockenen Form und in einer Menge vorliegen, die ausreichend ist, eine einzige viskoelastischen Analyse einer speziellen Blutprobe durchzuführen, und wobei die Bestandteile nicht in einem Substanzgemisch vorliegen, sondern jeweils in räumlich getrennten Pellets eingeschlossen sind;
c) Hinzufügen der Blutprobe zum Behälter (1), wodurch die darin enthaltene diagnostische Zusammensetzung gelöst wird;
d) Einbringen des Behälters (1) in ein Gerät (4), das zur Durchführung einer viskoelastischen Analyse geeignet ist; und
e) Durchführen der viskoelastischen Analyse des Gemisches.

2. Verfahren nach Anspruch 1, wobei die Blutprobe eine Säugetier-, besonders bevorzugt eine humane Blutprobe ist.

3. Verfahren nach Anspruch 1 oder 2, wobei Schritt c) ungefähr 1-60 Sekunden, vorzugsweise ungefähr 2-10 Sekunden, besonders bevorzugt ungefähr 5 Sekunden beträgt.

4. Verfahren nach einem oder mehreren der vorhergehenden Ansprüche, wobei das Gerät (4) ein Thromboelastometer oder ein Thromboelastograph ist.

5. Verfahren nach einem oder mehreren der vorhergehenden Ansprüche, wobei die Analyse die Bestimmung der Gerinnungszeit, der Gerinnselbildungszeit, der Festigkeit des Gerinnsels über die Zeit hinweg und/oder die Fibrinolyse umfasst.

6. Diagnostische Zusammensetzung zur Verwendung im Verfahren nach einem oder mehreren der Ansprüche 1-5, zur viskoelastischen Analyse einer Blutprobe, umfassend:
a) wenigstens einen Aktivator der Koagulation;
b) ein Kalziumsalz, vorzugsweise CaCl₂, in einer Menge die ausreichend ist, um die Rekalzifizierung der Blutprobe sicherzustellen; und
c) einen Heparin-Inhibitor;
**dadurch gekennzeichnet, dass**
die Bestandteile in einer im Wesentlichen trockenen Form und in einer Menge vorliegen, die ausreichend ist, um eine einzige viskoelastische Analyse einer speziellen Blutprobe durchzuführen, und wobei die Bestandteile nicht in einem Substanzgemisch vorliegen, sondern jeweils in räumlich getrennten Pellets eingeschlossen sind.

7. Zusammensetzung nach Anspruch 6, wobei die räumliche Trennung der Bestandteile durch Einbauen jedes Bestandteils in ein getrenntes Trägermaterial realisiert wird, wobei das Trägermaterial vorzugsweise wenigstens ein Kohlenhydrat, besonders bevorzugt Saccharose, Lactose oder Zellulose umfasst.

8. Diagnostische Zusammensetzung nach Anspruch 6, wobei der Aktivator der Koagulation ein intrinsischer und/oder extrinsischer Aktivator ist.

9. Diagnostische Zusammensetzung nach Anspruch 8, wobei der extrinsische Aktivator der Koagulation der Gewebefaktor (tissue factor = TF) ist, vorzugsweise lipidierter Gewebefaktor oder rekombinanter Gewebefaktor (rTF), oder
wobei der intrinsische Aktivator der Koagulation aus der Gruppe ausgewählt ist, die aus Celit, Ellaginsäure, Sulfatit, Kaolin, Silika, RNA, oder Mischungen hiervon besteht.

10. Diagnostische Zusammensetzung nach Anspruch 6, wobei der Heparin-Inhibitor aus Heparinase, Protamin oder Protaminverwandten Peptiden ausgewählt ist.

11. Diagnostische Zusammensetzung nach einem oder mehreren der Ansprüche 6-10, wobei der Koagulationsfaktor aus einem oder mehreren Koagulationsfaktoren oder aktivierten Koagulationsfaktoren ausgewählt ist, vorzugsweise FXa oder FVa oder aktiviertes Protein C oder FVIIa, und/oder wobei CaCl₂ in einer Menge von ungefähr 1-100 µmol/ml der Blutprobe vorliegt.

12. Diagnostische Zusammensetzung nach einem oder mehreren der Ansprüche 7-12, wobei die trockene Form eine lyophilisierte Form ist, und/oder
die weiterhin einen Stabilisator, vorzugsweise Albumin oder Gelatine umfasst.

13. Behälter, umfassend die diagnostische Zusammensetzung nach einem oder mehreren der Ansprüche 6-12, wobei der Behälter (1) vorzugsweise die Form eines Vials oder Küvette aufweist, und besonders bevorzugt in einer Art und Weise ausgebildet ist, dass sich das innere Seitenprofil von der Öffnung zum Boden hin reduziert.

14. Behälter nach Anspruch 13, dessen Innenanteil in einer Art und Weise ausgeformt ist, dass er an eine Vorrichtung zur Durchführung von viskoelastischen Messungen befestigt werden kann.

## Revendications

1. Procédé in vitro de réalisation d'une analyse viscoélastique sur un échantillon sanguin, comprenant les étapes de :
a) fournir un échantillon sanguin ;
b) fournir un récipient (1) contenant une composition de diagnostic comprenant au moins un activateur de coagulation ; un sel de calcium, de préférence CaCl₂, en une quantité suffisante pour assurer la recalcification de l'échantillon sanguin ; et un inhibiteur d'héparine ; dans lequel les constituants sont présents sous une forme essentiellement anhydre et en une quantité suffisante pour réaliser une simple analyse viscoélastique d'un échantillon sanguin spécifié, et dans lequel les constituants ne sont pas présents en un mélange de substances, mais sont chacun incorporés dans des pastilles spatialement séparées ;
c) ajouter l'échantillon sanguin dans ledit récipient (1), diluant ainsi la composition de diagnostic contenue à l'intérieur ;
d) mettre le récipient (1) dans un appareil (4) adapté pur réaliser une analyse viscoélastique ; et
e) réaliser l'analyse viscoélastique dudit mélange.

2. Procédé selon la revendication 1, dans lequel l'échantillon sanguin est un échantillon sanguin de mammifère, plus préferentiellement humain.

3. Procédé selon les revendications 1 ou 2, dans lequel l'étape c) prend environ 1 à 60 secondes, de préférence environ 2 à 10 secondes, plus préferentiellement environ 5 secondes.

4. Procédé selon l'une quelconque des revendications précédentes, dans lequel l'appareil (4) est un thromboélastomère ou un thromboélastographe.

5. Procédé selon une ou plusieurs des revendications précédentes, dans lequel l'analyse comprend la détermination du temps de coagulation, le temps de formation du caillot, la fermeté du caillot au cours du temps et/ou la fibrinolyse.

6. Composition de diagnostic pour une utilisation dans le procédé d'une ou plusieurs des revendications 1 à 5 pour l'analyse viscoélastique d'un échantillon sanguin, comprenant :
a) au moins un activateur de coagulation ;
b) un sel de calcium, de préférence CaCl₂, en une quantité suffisante pour assurer la recalcification de l'échantillon sanguin ; et
c) un inhibiteur d'héparine ;
**caractérisé en ce que**
les constituants sont présents en une forme essentiellement anhydre et en une quantité suffisante pour réaliser une simple analyse viscoélastique d'un échantillon sanguin spécifié, et dans laquelle les constituants ne sont pas présents en un mélange de substances, mais sont chacun incorporés dans des pastilles séparées spatialement.

7. Composition selon la revendication 6, dans laquelle la séparation spatiale des constituants est réalisée en incorporant chaque composant dans un matériau de support séparé, dans laquelle le matériau de support comprend de préférence au moins un carbohydrate, plus préferentiellement du saccharose, du lactose ou de la cellulose.

8. Composition de diagnostic selon la revendication 6, dans laquelle l'activateur de coagulation est un activateur intrinsèque et/ou extrinsèque.

9. Composition de diagnostic selon la revendication 8, dans laquelle le facteur extrinsèque de coagulation est le facteur tissulaire (FT), de préférence un TF lipidé ou un TF recombinant, ou
dans laquelle le facteur intrinsèque de coagulation est choisi dans le groupe constitué par la célite, l'acide ellagique, la sulfatite, le kaolin, la silice, un ARN ou leurs mélanges.

10. Composition de diagnostic selon la revendication 6, dans laquelle l'inhibiteur d'héparine est choisi parmi l'héparinase, la protamine ou des peptides apparentés à la protamine.

11. Composition de diagnostic selon l'une ou plusieurs des revendications 6 à 10, dans laquelle le facteur de coagulation est choisi parmi un ou plusieurs facteurs de coagulation ou des facteurs de coagulation activés, de préférence FXa ou FVa ou la protéine C activée ou FVIIa, et/ou
CaCl₂ étant présent en une quantité d'environ 1 à 100 µmoles/ml de l'échantillon sanguin.

12. Composition de diagnostic selon une ou plusieurs des revendications 7 à 12, dans laquelle la forme anhydre est une forme lyophilisée, et/ou
qui comprend en outre un stabilisateur, de préférence de l'albumine ou de la gélatine.

13. Récipient, comprenant la composition de diagnostic selon une ou plusieurs des revendications 6 à 12, dans lequel le récipient (1) prend de préférence la forme d'une fiole ou d'une cuvette, plus préferentiellement est formé d'une manière où le profil latéral interne se réduit de l'ouverture au fond.

14. Récipient selon la revendication 13 avec sa partie interne formée d'une manière par laquelle il peut être attaché à un dispositif pour réaliser des mesures viscoélastiques.
